# EUROPÄISCHE PATENTSCHRIFT

(11) **EP 1 176 984 B1**
(45) Veröffentlichungstag und Bekanntmachung des Hinweises auf die Patenterteilung: **10.01.2007**
(21) Anmeldenummer: 00927163.6
(22) Anmeldetag: 08.05.2000
(51) Int. Cl.: A61K 47/48, A61K 47/44, A61K 9/10, A61K 9/14, A61K 31/19, A61K 38/00

(54) **ARZNEISTOFFTRÄGER ZUR KONTROLLIERTEN WIRKSTOFFAPPLIKATION HERGESTELLT AUS LIPIDMATRIX-ARZNEISTOFF-KONJUGATEN (LAK-PARTIKEL)**
MEDICAMENT VEHICLE FOR THE CONTROLLED ADMINISTRATION OF AN ACTIVE AGENT, PRODUCED FROM LIPID MATRIX-MEDICAMENT CONJUGATES
EXCIPIENT POUR L'ADMINISTRATION CONTROLEE DE PRINCIPE ACTIF, PRODUIT A PARTIR DE CONJUGUES MATRICE LIPIDIQUE-SUBSTANCE PHARMACEUTIQUE

(30) Priorität: 07.05.1999 DE 19920908; 27.12.1999 DE 19964085
(43) Veröffentlichungstag der Anmeldung: 06.02.2002
(73) Patentinhaber: PharmaSol GmbH, 12307 Berlin (DE)
(72) Erfinder: MÜLLER, Rainer, Helmut, D-12161 Berlin (DE); OLBRICH, Carsten, D-10967 Berlin (DE)
(74) Vertreter: UEXKÜLL & STOLBERG
(86) Internationale Anmeldenummer: PCT/EP2000/004111
(87) Internationale Veröffentlichungsnummer: WO 2000/067800

(56) Entgegenhaltungen:
- WO-A-95/32002
- WO-A-96/14830
- WO-A-96/22773
- US-A- 5 554 728
- CHEMICAL ABSTRACTS, vol. 121, no. 7, 15. August 1994 (1994-08-15) Columbus, Ohio, US; abstract no. 073228g, CHEN Z ET AL: "Tributyrin: A prodrug of butyric acid for potential clinical application in differentiation therapy" XP002901400 & CANCER RES,, Bd. 54, Nr. 13, 1994, Seiten 3494-3499,
- AHLIN P ET AL: "Optimization of procedure parameters and physical stability of solid lipid nanoparticles in dispersions" ACTA PHARM, Nr. 48, 29. September 1998 (1998-09-29), Seiten 259-167, XP002901401
- CHEMICAL ABSTRACTS, vol. 121, no. 2, 11. Juli 1994 (1994-07-11) Columbus, Ohio, US; abstract no. 17931u, SCRIBA G: "Synthesis and in vitro evaluation of 4-(2-glyceryl)butyric acid: a glyceride mimic for drug delivery via drug-lipid cojugates" XP002901402 & ARCH. PHARM. , Bd. 327, Nr. 5, 1994, Seiten 347-348, Weinheim, DE

## Beschreibung

Ein Mittel zur Erzielung einer kontrollierten Arzneistoffapplikation ist der Einsatz von partikulären Trägern mit einer Partikelgröße im Mikrometerbereich oder im Nanometerbereich. Der Arzneistoff ist in den Träger inkorporiert, Beispiele sind O/W-Emulsionen, Liposomen, Polymermikropartikel, Polymernanopartikel, feste Lipidnanopartikel, Arzneistoffmikropartikel und Arzneistoffnanopartikel (Nanokristalle, Nanosuspensionen) (R. H. Müller, G. E. Hildebrand, Pharmazeutische Technologie: Moderne Arzneiformen, Wissenschaftliche Verlagsgesellschaft Stuttgart) oder auf den Träger über ein spaltbares Linkermolekül aufgebracket (WO95/32002). Das Hauptziel für den Einsatz partikulärer Trägersysteme ist neben der Reduktion von Nebenwirkungen die Einstellung eines optimierten Arzneistoffliberationsprofils. In der Regel strebt man eine gleichmäßig anhaltende (sustained) oder zumindest eine verlängerte (prolonged) Freisetzung an. Hohe initiale Freisetzung (sogenannter burst release) ist unerwünscht. Klassisches Beispiel hierfür sind Polymermikropartikel mit LHRH-Analoga zur Therapie des Prostatakarzinoms mit Freisetzungsdauer über vier Wochen (Handelsprodukte: Decapeptyl, Enantone).

Ein ernsthaftes Problem, das in vielen Fällen nicht gelöst werden kann, ist eine bei der Einarbeitung von Arzneistoffen in diese Arzneistoffträger auftretende hohe initiale Freisetzung. Emulsionen sind in der Regel nicht für eine prolongierte Freisetzung geeignet, da der in den Emulsionstropfen gelöste Wirkstoff sich bei Verdünnen (z. B. Injektion ins Blut) innerhalb von Millisekunden aus dem Blut in die wäßrige Phase umverteilt (C. Washigton, in (R.H. Müller, S. Benita, B. Böhm, Hrsg.) Emulsions and Nanosuspensions for the Formulation of Poorly Soluble Drugs, medpharm scientific publishers Stuttgart, 101-117, 1998). Eine prolongierte Freisetzung aus Liposomen ist nur beschränkt möglich, da identische Umverteilungsprozesse des Wirkstoffes und die Metabolisierung der Phospholipide der Liposomen die Freisetzungszeit limitieren. Nur bei geeigneter Herstellungstechnik erhält man mit Polymermikropartikeln eine ausreichend prolongierte Freisetzung (z. B. Decapeptyl), bei ungeeigneter Herstellungstechnik wie dem ASES (Aerosol Solvent Extraction System) (B.W. Müller et al., US Patent No. 5043.280 (1991)) wird eine sehr hohe initiale Freisetzung erhalten. Erzielung einer prolongierten Freisetzung ist noch schwieriger bei Nanopartikeln, da aufgrund der Kleinheit der Partikel die Diffusionsstrecken sehr kurz sind und die Abbaugeschwindigkeit teilweise sehr schnell. Arzneistofffreisetzung erfolgt schlagartig aufgrund von Diffusion, was sowohl bei Polymernanopartikeln als auch bei festen Lipidnanopartikeln beobachtet wurde (zur Mühlen, A. et al. Eur. J. Pharm. Biopharm. 1998, 45, 149-155). Speziell bei schwerlöslichen Arzneistoffen kann man mit Zerkleinerungsverfahren Mikropartikel aus reinem Wirkstoff herstellen. Aufgrund der geringen Wasserlöslichkeit der Wirkstoffe (generell verbunden mit einer geringen Auflösungsgeschwindigkeit) in Kombination mit der relativ geringen Partikeloberfläche kommt es zu einem verlangsamten Freisetzungprozeß. Beispiele sind Corticoid-Mikropartikelsuspensionen zur intramuskulären oder intraartikulären Injektion. Für einige Anwendungsgebiete wäre jedoch eine längere Freisetzungszeit wünschenswert. Durch hochenergetisches Mahlen kann man schwerlösliche Arzneistoffe zu Nanopartikeln zerkleinern (Nanokristalle, in wäßriger Dispersion als Nanosuspensionen bezeichnet (Müller, R.H. et al., Pharm.Ind. 1999, 61, 1, 74-78). Aufgrund der stark vergrößerten Oberfläche kommt es bei Nanokristallen aber zu einer sehr schnellen Auflösung. Intravenös injizierte Nanosuspensionen verhielten sich pharmakokinetisch wie eine Lösung (z. B. Cyclosporin) (H. Sucker, in Pharmazeutische Technologie: Moderne Arzneiformen (R.H. Müller, G.E. Hildebrandt, Hrsg.), Wissenschaftliche Verlagsanstalt Stuttgart, 383-391, 1998).

Mikropartikel mit einer Größe im unteren Mikrometerbereich und Nanopartikel besitzen jedoch in der Literatur beschriebene Vorteile für die Arzneistoffapplikation. So zeigen sie nach peroraler Applikation eine Adhäsion an die Magen-Darm-Schleimhaut. Als Folge erhöht sich die Bioverfügbarkeit, gleichzeitig nimmt die Variabilität ab. Aufgrund der Partikelfeinheit können schwerlösliche Arzneistoffe, die nach oraler Applikation keine ausreichende Bioverfügbarkeit zeigen, intravenös injiziert werden (R.H. Müller, in Pharmazeutische Technologie: Moderne Arzneiformen (R.H. Müller, G.E. Hildebrand, Hrsg.) Wissenschaftliche Verlagsanstalt Stuttgart, 393-400, 1998). Somit erreicht man auch bei schwerlöslichen Arzneistoffen eine ausreichend hohe Bioverfügbarkeit. Aufgrund dieser Vorteile wäre es wünschenswert, feine Partikel herstellen zu können, bei denen die rasche Freisetzung aufgrund von Wirkstoff-Diffusion eliminiert oder zumindest minimiert ist.

In der vorliegenden Erfindung wird dies dadurch erreicht, daß der Arzneistoff durch kovalente Bindungen, elektrostatische Wechselwirkungen, Dipol-Dipol-Wechselwirkungen, Dispersionskräfte, Ionenwechselwirkungen, Wasserstoffbrücken und/oder hydrophobe Wechselwirkungen an das Matrixmaterial der Partikel gebunden wird.

Es kann gemäß einigen erfindungsgemäßen Ausführungsformen nur eine dieser Bindungsarten vorliegen, oder es können gemäß anderen Ausführungsformen der Erfindung auch mehrerere dieser Bindungsarten gemeinsam vorliegen.

Es gibt somit
a) Ausführungsformen mit kovalenten Bindungen,
b) Ausführungsformen mit nicht-kovalenten Bindungen und
c) Ausführungsformen mit einem Anteil an kovalenten und einem Anteil an nicht-kovalenten Bindungen

Insbesondere bei den Ausführungsformen mit nicht-kovalenten Bindungen in Form von elektrostatischen Wechselwirkungen, Dipol-Dipol-Wechselwirkungen, Dispersionskräften, Ionenwechselwirkungen, Wasserstoffbrücken und/oder hydrophoben Wechselwirkungen kann gemäß obigem c) auch ein Anteil an kovalenter Bindung vorliegen.

Als Matrixmaterial werden Lipide eingesetzt.

In der Literatur sind bereits Konjugate aus Arzneistoffen bzw. Prodrugs mit Lipiden beschrieben, wobei hier die Zielstellung war, durch Kopplung eines Wirkstoffes mit einer lipophilen Komponente die Membrangängigkeit und damit die Arzneistoffabsorption zu erhöhen. Voraussetzung für eine gute Absorption sind jedoch neben Membrangängigkeit auch eine ausreichend hohe Löslichkeit in Wasser. Es nützt nichts, wenn ein derartiges Konjugat zwar sehr lipophil ist gleichzeitig aber gering wasserlöslich. Aufgrund der niedrigen Wasserlöslichkeit kommt in diesem Fall zu wenig Arzneistoff an die Membran. Lösungsgeschwindigkeit und Wasserlöslichkeit werden dann zum geschwindigkeitsbestimmenden Schritt der Absorption. Um dies zu verhindern, war bei diesen Lipid-Prodrug-Konjugaten das Ziel, Konjugate mit möglichst hoher Wasserlöslichkeit herzustellen. In der vorliegenden Erfindung ist gerade das Umgekehrte der Fall, die Wasserlöslichkeit soll möglichst gering sein, um eine initiale Freisetzung zu minimieren. Arzneistoff soll anstatt durch Diffusion durch Abbau freigesetzt werden, d. h. nach chemischer Aufspaltung des Konjugates (z. B. durch Enzyme im Magen-Darm-Trakt oder in anderen Körperflüssigkeiten wie Blut).

In der vorliegenden Erfindung werden nicht nur durch kovalente Bindungen stabile Konjugate erzeugt, sondern auch die durch nicht-kovalente Bindung erzeugten Konjugate sind trotz Abwesenheit von kovalenten Bindungskräften so stabil sind, daß daraus Partikel hergestellt werden können.

Polymer-Arzneistoff-Konjugate zeigen oft das Problem, daß sie als unphysiologische Komponenten im Organismus nicht oder nur langsam aufgespalten werden. Abspaltung des Arzneistoffes vom Polymer (sogenannte Cleavage) ist aber Voraussetzung für Freisetzung und Wirksamkeit. Zur Erzielung einer besseren Abbaubarkeit in vivo werden daher in der vorliegenden Erfindung Lipide als Matrixmaterial eingesetzt. Toxikologisch besteht zusätzlich der Vorteil, daß nach Spaltung des Konjugates der Lipidanteil verstoffwechselt werden kann. Er dient gleichzeitig als Nahrungsstoff.

Bei den Lipid-Prodrugs mit noch vorhandener entsprechender Wasserlöslichkeit erfolgt der Abbau des Moleküls in Lösung. Bei den in dieser Erfindung beschriebenen unlöslichen Partikeln wurde gefunden, daß das Lipid-Konjugat trotz seines festen Aggregatzustandes abgebaut werden kann. Dies erfolgt durch Verankern von Enzymkomplexen auf der Partikeloberfläche, es findet eine Oberflächendegradation statt, bei der die Arzneistoffmoleküle freigesetzt werden (z. B. Anlagerung des Lipase/Colipase-Komplexes im Gastrointestinaltrakt). Die bessere Abbaubarkeit von Lipid-Arzneistoff-Konjugaten im Vergleich zu Polymer-Arzneistoff-Konjugaten kann dadurch erklärt werden, daß z. B. die Lipid-abbauenden Enzyme im Organismus aufgrund der chemischen Vielfalt der Lipide in der Nahrung teilweise sehr unspezifisch ausgelegt sind. Daher können auch Lipid-Arzneistoff-Konjugate entsprechend prozessiert werden. Demgegenüber gehören Polymere wie z. B. Polymethacrylate und Polyhydroxybutyrate (PHB) nicht zum menschlichen Nahrungsangebot. PHB ist zwar das Engergie-Speicherpolymer von Bakterien, nicht jedoch vom Menschen in vivo abbaubar.

Die Partikelmatrix der erfindungsgemäßen Arzneistoffträger besteht zu 100 % aus Lipid-Arzneistoff-Konjugat (Lipid-Arzneistoff-Konjugat - LAK) (Beispiel 1).

Die Herstellung von LAK-Partikeln mit kovalenter Bindung erfolgt z.B. durch Dispergierung oder Präzipitation, wobei in Lehrbüchern der Pharmazie und Verfahrenstechnik beschriebene allgemein bekannte Methoden eingesetzt werden. Bei der Dispergierung zerteilt man grobdisperse Lipide durch mechanische Verfahren. Die Lipide können sich hierbei im festen Aggregatzustand (z. B. Mörsermühle) oder im flüssigen Aggregatzustand befinden (z.B. Emulgierung geschmolzener Lipide durch Rührer). Zur Herstellung der LAK-Dispersion können die Lipide zuerst zerkleinert und anschließend in der äußeren (z.B. wäßrigen) Phase dispergiert werden oder alternativ direkt in der äußeren Phase zerkleinert werden. Zur Erzeugung von hochfeinen Partikeln im Größenbereich 1 - 10 µm und insbesondere im Nanometerbereich (<1000 nm) eignen sich insbesondere Hochdruckhomogenistionsverfahren (Kolben-Spalt-Homogenisatoren, Jet-Stream-Hochdruckhomogenistoren wie z. B. Microfluidizer) und Rotor-Stator-Kolloidmühlen wobei hierbei das grobdisperse Matrixmaterial in einer Flüssigkeit dispergiert ist (z. B. Wasser, nichtwäßrige Medien wie Polyethylenglykol 400/600 und Öle wie Miglyole). In flüssiger Dispersion sind die Arzneistoffträger durch Tenside oder Polymere physikalisch stabilisiert. In Dispersionsmedien mit ausreichend hoher Viskosität sind keine Stabilisatoren erforderlich (Tensid-freie Dispersionen). Die erfindungsgemäßen Arzneistoffträger können auch in einer festen Dispersion vorliegen, d. h. die Arzneistoffträger sind in einer festen äußeren Phase eingelagert, z. B. Polyethylenglykol 10000.

Anstatt vollständig aus Lipid-Arzneistoff-Konjugat (LAK) zu bestehen, kann der Matrix der erfindungsgemäßen Arzneistoffträger auch ein Lipid zugesetzt sein, d.h. aus einer Mischung von LAK mit einem oder mehreren Lipiden bestehen. Ein Beispiel ist die Mischung des LAK Behenylalkohol-Buttersäure-Ester mit Cetylpalmitat (Beispiel 2) oder des LAK Tributyrin mit Compritol (Triglycerid der Behensäure) (Beispiel 3). Dies ist insbesondere dann empfehlenswert, wenn eine raschere Arzneistofffreisetzung wünschenswert ist und der Abbau des LAK beschleunigt werden soll. Zusatz eines schnell abbaubaren Lipids wie Cetylpalmitat führt nach seinem Abbau zur Vergrößerung der Oberfläche und daraus resultierend schnellerem Abbau der LAK-Partikel.

Die Herstellung eines Lipid-Konjugates mit nicht-kovalenten Bindungen erfolgt z.B. durch Aufschmelzen der konjugatbildenden Komponenten (Schmelzmethode) oder durch Lösen der Komponenten in einem gemeinsamen Lösungsmittel und anschließendem Abdampfen des Lösungsmittels (Lösungsmethode). Wirkstoff und konjugatbildene Komponente zeichnen sich dadurch aus, daß die Moleküle gegensätzlich geladene Gruppen bestzen (z.B. quartäre Ammoniumgruppe und dissoziierte Carboxygruppe) oder Molekülteile, die nicht kovalente Wechselwirkungen miteinander Ausbilden (z.B. hydrophobe Wechselwirkungen). Beispiele für Arzneistoffe mit einer primären Amino- oder Guanidinfunktion sind Diminazen, SISPI, Pentamidin, Melarsoprol, Cisplatin und Hydroxyharnstoff.

So werden z.B. bei der Schmelzmethode Wirkstoff und gegensätzlich geladene zweite Konjugatkomponente (z.B. Diminazen und Stearinsäure im molaren Verhältnis 1:2, Beipiel 5) gemischt und erhitzt, anschließend abgekühlt und es hat sich das Konjugat gebildet.

Bei der Lösungsmethode werden beide Komponenten in einem wäßrigen oder nichtwäßrigen Lösungsmittel aufgelöst (z.B. Diminazen und Stearinsäure, molares Verhältnis 1:2 in Ethanol, Beispiel 6) und erhitzt. Nach Evaporation des Ethanols erhält man als Rückstand das Konjugat. Als Lösungsmittel können z.B. Wasser, Alkohole, Öle, flüssige Polyethylenglykole (PEG) oder auch durch Erhitzen verflüssigte Komponenten (z.B. bei Raumtemperatur feste PEG oder Lipide wie Imwitor 900) sowie deren Mischungen eingesetzt werden.

Eine Vielzahl unterschiedlicher Lipide kann zur Herstellung von LAK-Dispersionen eingesetzt werden. Dies sind sowohl chemisch einheitliche Lipide als auch ihre Mischungen. Charakterisiert sind die Lipide dadurch, daß sie im Endprodukt LAK-Dispersion im kristallinen Zustand (z.B. ß-, ßi-Modifikation) oder im flüssigkristallinen Zustand (α-Modifikation) vorliegen bzw. in deren Mischung. Bei eingesetzten Lipidmischungen können auch flüssige Lipide (z. B.Öle, lipophile Kohlenwasserstoffe, lipophile organische Flüssigkeiten wie Oleylalkohol) den festen Lipiden (z.B. Glyceride, lipophile Kohlenwasserstoffe wie Hartparaffin) zugemischt werden (sog. "lipid blends").

Einsatz finden z. B. folgende Lipide als dispergierte Phase und können als individuelle Komponente oder als Mischung angewendet werden: Natürliche oder synthetische Triglyceride bzw. Mischungen derselben, Monoglyceride und Diglyceride, alleine oder Mischungen derselben oder mit z.B. Triglyceriden, selbst-emulgierende modifizierte Lipide, natürliche und synthetische Wachse, Fettalkohole, einschliesslich ihrer Ester und Ether sowie in Form von Lipidpeptiden, oder irgendwelche Mischungen derselben. Besonders geeignet sind synthetische Monoglyceride, Diglyceride und Triglyceride als individuelle Substanzen oder als Mischung (z.B. Hartfett), Imwitor 900, Triglyceride (z.B. Glyceroltrilaurat, Glycerolmyristat, Glycerolpalmitat, Glycerolstearat und Glycerolbehenat) und Wachse wie z.B. Cetylpalmitat und weisses Wachs (DAB).

Zur Stabilisierung der LAK-Dispersionen oder zu ihrer gezielten Oberflächenmodifikation können die Tenside, Stabilisatoren und Polymere eingesetzt werden, die allgemein aus der Herstellung von Dispersionen bekannt sind. Beispiele dafür sind:
1. sterisch stabilisierende Substanzen wie Poloxamere und Poloxamine (Polyoxyethylen-Polyoxypropylen-Block-Copolymere), ethoxylierte Sorbitanfettsäure-Ester, besonders Polysorbate (z.B. Polysorbat 80 bzw. Tween 80®), ethoxylierte Mono- und Diglyceride, ethoxylierte Lipide, ethoxylierte Fettalkohole oder Fettsäuren, und Ester und Ether von Zuckern oder von Zuckeralkoholen mit Fettsäuren oder Fettalkoholen (z.B. Saccharose-Monostearat);
2. geladene ionische Stabilisatoren so wie Diacetylphosphate, Phosphatidylglycerin, Lecithine unterschiedlicher Herkunft (z.B. Eilecithinoder Sojalecithin), chemisch modifizierte Lecithine (z.B. hydrierte Lecithine), genauso wie Phospholipide und Sphingolipide, Mischung von Lecithinen mit Phospholipiden, Sterolen (z.B. Cholesterol und CholesterolDerivate, genauso wie Stigmasterin) und ebenfalls gesättigte und ungesättigte Fettsäuren, Natriumcholat, Natriumglycocholat, Natriumtaurocholat, Natriumdeoxycholat oder ihrer Mischungen, Aminosäuren oder Anti-Flokkulantien, wie z.B. Natriumcitrat, Natriumpyrophosphat, Natriumsorbat [Lucks, J.S. et al. Int. J. Pharm., 1990, 58, 229 - 235]. Zwitterionische Tenside wie z.B. (3-[(3-cholamidopropyl)-dimethylammonio]-2-hydroxy-1-propanesulfonate) [CHAPSO], (3-[(3-cholamidopropyl)-dimethylammonio]-1-propanesulfonate) [CHAPS] und N-dodecyl-N,N-dimethyl-3-ammonio-lpropansulfonat. Kationische Tenside, z.B. Benzyldimethylhexadecylammoniumchlorid, Methylbenzethoniumchlorid, Benzalkoniumchlorid, Cetylpyridiniumchlorid.
3. Viskositätserhoehende Substanzen wie z.B. Cellulose-Ether und Cellulose-Ester (z.B. Methylcellulose, Hydroxyethylcellulose, Hydroxypropylcellulose, Natriumcarboxymethylcellulose), Polyvinylderivate sowie Polyvinylalkohol, Polyvinylpyrrolidon, Polyvinylacetat, Alginate, Polyacrylate (z.B.Carbopol), Xanthane und Pektine.

Die geladenen Stabilisatoren sind, wenn notwendig oder gewünscht, vorzugsweise mit 0,01% bis 20% (m/m) und insbesondere in einer Menge von 0,05% bis zu 10% in der LAK-Dispersion enthalten. Viskositätserhöhende Substanzen sind, wenn notwendig oder erwünscht, im ähnlichen Verhältnis in der Formulierung eingearbeitet, vorzugsweise in einer Menge von 0,01-20% und insbesondere in einer Menge von 0,1% bis 10% (m/m) und vorzugsweise im Bereich zwischen 0,5% und 5%.

Als äußere Phase (Dispersionsmedium, kontinuierliche Phase) können Wasser, wässrige Lösungen oder Flüssigkeiten mischbar mit Wasser, sowie Glycerin oder Polyethylenglykol und ölige Flüssigkeiten wie Miglyole (medium chain triglycerides - MCT) und andere Öle (Rizinus-, Erdnuß-, Soja-, Baumwollsamen-, Raps-, Leinsamen-, Oliven-, Sonnenblumen-, Distelöl eingesetzt werden.

Tensidfreie LAK werden hergestellt durch Dispergierung der Lipidphase in einer wäßrigen Lösung, die eine oder mehrere viskositätserhöhende Substanzen enthält, entweder allein oder in Kombination mit anderen Substanzen, sowie Zucker, Zuckeralkohole, besonders Glukose, Mannose, Trehalose, Mannitol, Sorbitol sowie andere. Desweiteren ist es möglich, eine Kombination der viskositätserhöhenden Stoffe oder die Kombination dieser mit Zuckern oder Zuckeralkoholen, oder in einer weiteren Kombination mit Ladungsstabilisatoren oder Anti-Flokkulantien zu gebrauchen.

Die Partikelmatrix kann auch aus einer Mischung von einem oder mehreren LAK und einem oder mehreren Arzneistoffen bestehen. Dies ist insbesondere dann einsetzbar, wenn vor einer prolongierten Freisetzung noch zusätzlich eine Initialdosis benötigt wird.

Die Partikelmatrix der erfindungsgemäßen Arzneistoffträger kann auch obige Prinzipien kombinieren, d. h. die Matrix besteht aus einer Mischung von LAK, einem oder mehreren Lipiden und einem oder mehreren Arzneistoffen.

In den LAK-Partikeln kann der Arzneistoff gekoppelt sein an unterschiedliche Lipide, z.B. ein oder mehrere Diglyceride, Monoglyceride, Glycerol, (z.B. Tributyrin), Fettsäuren, Fettalkohole (Z.B. Behenylalkohol-Buttersäure-Ester), funktionelle Gruppen von Sterolen wie Cholesterol und Cholesterolderivate und von Wachsen.

Die Bindung von Arzneistoffen kann dabei kovalent oder nichtkovalent über andere Wechselwirkungen (z.B. Ionenpaare) unterschiedlicher funktioneller Gruppen des Lipids erfolgen, z. B. Hydroxylgruppen, Carboxy-, primäre, sekundäre und quartäre Aminogruppen. Neben kovalenter Bindung kann gegebenenfalls auch ein Anteil nicht-kovalenter Bindung vorliegen, z. B. Ionenpaare (Vitamin C mit Lecithin), oder umgekehrt kann neben nicht-kovalenter Bindung gegebenenfalls auch ein Anteil kovalenter Bindung vorliegen.

Als Arzneistoffe oder Arzneistoffgruppen kommen erfindungsgemäß insbesonder in Frage:
► Buttersäure und insbesondere Behenylalkohol-Buttersäure-Ester und Buttersäure-Triglycerid (Tributyrin), und
► ß-Aminosäuren und Peptide und Proteine aus diesen Aminosäuren.

Beispiele für LAK mit kovalenter Bindung sind Tributyrin, Buttersäure-Derivate (z. B. 4-(2-glyceryl)butyric acid, Behenylalkohol-Buttersäure-Ester), Retinolpalmitat, Tocopherolpalmitat etc.

Tributyrin ist ein Prodrug der Buttersäure, das eine Zelldifferenzierung in vitro in einer großen Zahl von neoplastischen Zellen bewirkt. Der klinische Nutzen der Buttersäure ist bisher beschränkt aufgrund der Schwierigkeiten, effektive therapeutische Konzentrationen zu erreichen, aufgrund ihres schnellen Metabolismus' [Z.X. Chen et al. Cancer Res. 54 (1994) 3494-3499]. Eine Strategie, effective Buttersäurespiegel in vivo zu erreichen ist es, Buttersäureverbindungen als Prodrugs zu verwenden, die unter in vivo Bedingungen metabolisiert werden können, um effective Buttersäurekonzentrationen zu geben. Eine dieser Verbindungen ist Tributyrin, der 1,2,3 Buttersäure Glycerol Ester, der große biologische Aktivität in in vitro Versuchen gezeigt hat [Z.X. Chen et al. Cancer Res. 54 (1994) 3494-3499, C. Schröder et al. Int. J. Oncol. 13 (1998) 335-1340]. Tributyrin wurde in einer Phase 1 Studie als orale Zubereitung in der Behandlung solider Tumoren untersucht. Die Ergebnisse waren enttäuschend. Keine signifikante Tumorregression hatte stattgefunden. [B.A. Conley et al. Clin. Canc. Res. 4 (1998) 629-634]. Dies ist eine Folge des schnellen Metabolismus' des Tributyrins und der Buttersäure.

Als neues Konzept der Krebsbehandlung mittels Tributyrin, bzw der aktiven Verbindung Buttersäure haben feste Lipid Arzneistoff-Konjugate (LAK) entwickelt. Einmal Tributyrin in Mischung mit Compritol, Zum anderen ein Ester aus Behenylalkohol und Buttersäure. Die Formulierung als feste Partikel eröffnet die Möglichkeit zum einen einer retardierten Freisetzung des Wirkstoffes Buttersäure, zum anderen die Möglichkeit das Konzept des Drug Targetings, also der gezielten Wirkstoffapplikation in definierten Gebieten. So Ist es z.B. möglich, die LAK Partikel in einer Arzneiform für das Colon Delivery zu verarbeiten und damit lokal Kolonkarzinomerkrankungen zu behandeln. Weiterhin ist es möglich, die Partikel mit bestimmten Tensiden herzustellen, die eine definierte Anreicherung in bestimmten Körperregionen ermöglichen, z.B. Polobamer 407, Anreicherung im Knochenmark, bei Leukämieerkrankungen).

Bei Herstellung von LAK-Partikeln durch Naßmahlung des Partikelmatrixmaterials in geschmolzenem Polyethylenglykol (PEG) 10.000 (z.B. bei 80°C) verfestigt sich die äußere Phase bei Abkühlung auf Raumtemperatur. Es entsteht eine feste Dispersion, d.h. LAK-Partikel eingebettet in festes PEG 10.000. Diese kann z.B. gemahlen und als Pulver in Tabletten und Pellets verarbeitet oder in Hartgelatine-Kapseln gefüllt werden. Zur Abfüllung sowohl in Weich- als auch in Hartgelatinekapslen kann die feste Dispersion auch erneut geschmolzen und im flüssigen Zustand in die Kapseln gefüllt werden.

Herstellung von Trockenprodukten aus LAK-Dispersionen ist mit üblichen Verfahrenstechniken wie z.B. Sprühtrocknung, Lyophilisation, Walzentrocknung und Vakuumtrocknung möglich. Die Trockenprodukte können dann zu traditionellen Arzneiformen wie z.B.

Tabletten, Kapseln, Pellets, Sachets oder Trockenprodukte zu Rekonstitution (z.B. für Injektabilia) weiterverarbeitet werden.

### Beispiele

Beispiel 1: Herstellung von Arzneistoffträgern aus dem Lipid-Arzneistoff-Konjugat(LAK) Behenylalkohol-Buttersäure-Ester: Das LAK wurde wie folgt synthetisiert: Buttersäurechlorid wurde mit Behenylakohol in Dichlormethan in Gegenwart des Katalysators Dimethylaminopyridin (DMPA) bei Raumtemperatur umgesetzt und durch Umkristallisieren aus Aceton gereinigt. Zur Herstellung wurde das LDC bei 46° C geschmolzen und in einer wäßrigen Tensidlösung mit einem Rotor-Stator-Rührer dispergiert (Ultra-Turax, Firma Jahnke und Kunkel, Germany, 10000 Umdrehungen pro Minute, für 1 Minute). Die Tensidlösung bestand aus 5% Poloxamer 188 in Wasser. Die erhaltene Rohemulsion wurde dann in einem Hochdruckhomogenisator Micron LAB 40 bei 500 bar und 3 Zyklen homogenisiert. Die Partikelgrößenbestimmung erfolgte mit Photonenkorrelationsspektrospie (PCS) und Laserdiffraktometrie (LD, Volumenverteilung). Der PCS-Durchmesser war 149 nm, der Polydispersitätsindex betrug 0,196. Der LD-Durchmesser 90 % - als Maß für den Anteil an Mikrometerpartikeln betrug 0,39 µm, d. h. die Kontamination durch Mikrometerpartikel war äußerst gering.

Beispiel 2: Herstellung von Arzneistoffträgern aus dem LAK Behenylalkohol-Buttersäure- Ester in Lipidzumischung: Das LAK wurde wie in Beispiel 1 synthetisiert. Anschließend wurde 1 Teil LAK mit 1 Teile Cetylpalmitat gemischt, geschmolzen und dann wie in Beispiel 1 verarbeitet. Der PCS-Durchmesser betrug 203 nm, der Polydispersitätsindex 0,210.

Beispiel 3: Herstellung von Arzneistoffträgern aus Tributyrin-Lipidmischung: Tributyrin wurde mit dem Lipid Compritol im Verhältnis 3 Teile zu 7 Teilen gemischt und dann analog zu Beispiel 1 zu einer Lipidpartikel-Dispersion verarbeitet. Der mittlere PCS-Durchmesser betrug 268 nm, der Polydispersitätsindex 0,248.

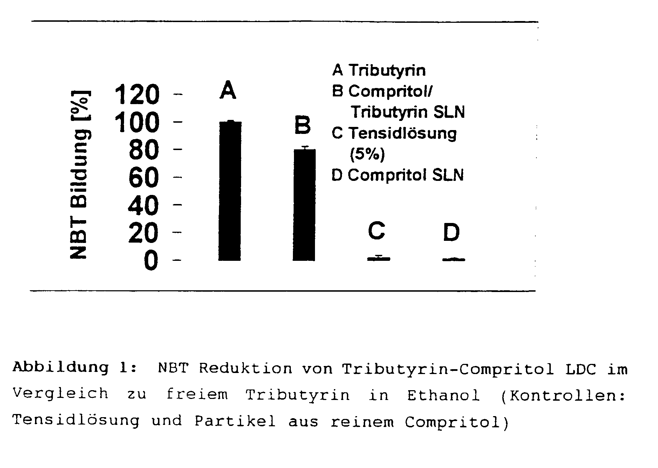

Beispiel 4: Die Wirkeffizienz von Tributyrin-Arzneistoffträgern wurde in HL 60 Tumorzellen (humane myeloide Leukämie Zellinie) bestimmt. HL 60 Zellen sind ein sensitiver Indikator für die Wirkung von Tributyrin. Es bewirkt eine Differenzierung der Tumorzellen hin zu granulozytenähnlichen Zellen. Diese haben die Eigenschaft, NBT (Nitro-Blue-Tetrazolium) zu einem blauen Farbstoff zu reduzieren (H.P. Koeffler et al. Blood 62 1(983)709-721). Tributyrin-Compritolpartikel, bestehend aus gleichen Teilen Tributyrin und Compritol (Glyceroltribehenat) wurden wie in Beispiel 1 hergestellt. Die gesamte Lipidkonzentration betrug 5%, die Poloxamer 188-Konzentration ebenfalls 5%. Die Partikelgröße war 147 nm (PCS Durchmesser), der Polydispersitätsindex 0,321. Die differenzierende Wirkung wurde verglichen mit der von freiem Tributyrin, gelöst in Ethanol. Die effektive Dosis 50% (ED 50%) betrug 130 µM. Eine adäquate Menge an Tributyrin-Compritol Nanopartikeldispersion wurde ebenso wie die entsprechenden Mengen an Tensidlösung und reiner Compritolpartikeldispersion zu HL 60 Zellen gegeben und nach 6 Tagen Inkubationszeit bei 37°C und 5% Kohlendioxidgehalt, die NBT Reduktion ermittelt. Figur 1 zeigt, daß die differenzierende Wirkung 80% der Wirkung von freiem Tributyrin entsprach.

Beispiel 5: Herstellung eines Diminazen-Stearinsäure-Konjugates über die Schmelzmethode:
Diminazen und Stearinsäure wurden im molaren Verhältnis 1:2 gemischt und erhitzt. Dabei entstand ein gelbes Konjugat.

Beispiel 6: Herstellung eines Diminazen-Stearinsäure-Konjugates über die Lösungsmethode: Diminazen und Stearinsäure wurden im molaren Verhältnis 1:2 in Ethanol gelöst und erhitzt. Nach vollständiger Lösung beider Komponenten wurde der Ethanol verdampft und als Rückstand eine gelbes Konjugat erhalten (identisch zu Beipiel 5).

Beispiel 7: Herstellung von LAK-Nanopartikel über Kalthomogenisation: Das Diminazen-Sterainsäure-Konjugat aus Beispiel 5 wurde mit Tensidlösung (1% Tween 80) angerieben und mit einem Hochdruckhomogenisator (Micron LAB 40, APV Deutschland GmbH, Lübeck, Deutschland) bei 15000 bar mit 15 Zyklen homogenisiert. Nach 15 Zyklen erhält man eine Dispersion von Diminazen-Stearinsäure-Nanopartikeln. Der mit Photonenkorrelationsspektroskopie (PCS, Malvern Zetasizer 4, Malvern Instruments, UK) bestimmte mittlere Durchmesser betrug 314 nm (Standardabweichung: 14 nm) mit einem Polydispersitätsindex (PI) von 0,214 (Standardabweichung 0,01). Der mit der Laserdiffraktometrie gemessene Durchmesser 95% betrug 0,693 µm (Coulter LS 230, Coulter Electronics, Deutschland).

Beipiel 8: Herstellung von LAK-Nanopartikeln mit der Lösungsmethode: Das Diminazen-Stearinsäure-Konjugat wurde in Ethanol 96% gelöst (2,5% m/m) und mit einem Perfusor (Braun Melsungen, Deutschland) mit einer Rate von 60 ml/h in 40 ml einer 1%igen Tween 80 Lösung gepumpt. Das verwendete Kanülensystem war Venofix S, 30 cm, 0,5mm - 25G. In die Lösung tauchte ein Ultraschallstab ein. Die Zeit des Zuflusses betrug 20 min. Die Partikelgröße wurde mit PCS und Laserdiffraktometrie bestimmt: PCS-Durchmesser 462 nm mit einem PI Wert von 0,255. Der LD-Durchmesser 95% betrug 0,488 µm.

Falls gewünscht, kann der Ethanol durch Evaporation entfernt werden. Nach Evaporation des Ethanols im Rotavapor betrugen der PCS-Durchmesser 456 nm und der PI Wert 0,275, d.h. Partikelgröße und Verteilung blieb unverändert.

Beispiel 9: Analog Beispiel 5 wurde Diminazen mit Ölsäure, einer flüssigen Fettsäure umgesetzt.

Beispiel 10: Das Diminazen-Ölsäure-Konjugat aus Beispiel 9 wurde analog Beispiel 7 hochdruckhomogenisiert. Die erhaltenen Konjugat-Nanopartikel hatten einen PCS-Durchmesser von 472 nm und einen PI von 0,268. Der LD-Durchmesser 95% Wert betrug 0,707 µm.

Beispiel 11: Analog Beispiel 5 wurde SISPI als Arzneistoff eingesetzt und wie in Beispiel 7 mit Hochdruckhomogenisation zu Nanopartikeln verarbeitet. Der PCS- Durchmesser betrug 421 nm, der Polydispersitätsindex betrug 0,207.

## Patentansprüche

1. Partikuläre Wirkstoffträger, die sich bei Raumtemperatur (20°C) im festen Aggregatzustand befinden, bestehend aus einem reinen Lipid-Arzneistoff-Konjugat (LAK) oder einer Mischung aus mehreren LAK als Partikelmatrix, wobei die Bindung im LAK durch kovalente Bindung, elektrostatische Wechselwirkungen, Dipol-Dipol-Wechselwirkungen, Dispersionskräfte, Ionenwechselwirkungen, Wasserstoffbrücken und/oder hydrophobe Wechselwirkungen bewirkt wird.

2. Partikuläre Wirkstoffträger nach Anspruch 1, bei denen zusätzlich zu elektrostatischen Wechselwirkungen, Dipol-Dipol-Wechselwirkungen, Dispersionskräften, Ionenwechselwirkungen, Wasserstoffbrücken und/oder hydrophoben Wechselwirkungen auch ein Anteil an kovalenter Bindung vorliegt.

3. Partikuläre Wirkstoffträger nach Anspruch 1 oder 2 mit einer mittleren Partikelgröße im Bereich von 10-1000 Nanometern.

4. Partikuläre Wirkstoffträger nach Anspruch 1 oder 2 mit einer mittleren Partikelgröße im Bereich von 10-1000 Mikrometern.

5. Partikuläre Wirkstoffträger nach einem der Ansprüche 1 bis 4, bei denen der LAK-Matrix ein oder mehrere Lipide zugemischt sind.

6. Partikuläre Wirkstoffträger nach Anspruch 5, bei denen in der Partikelmatrix, bezogen auf das Gesamtgewicht, 0,1% bis zu 50% zugemischtes Lipid enthalten sind.

7. Partikuläre Wirkstoffträger nach Anspruch 5, bei denen in der Partikelmatrix, bezogen auf das Gesamtgewicht, von 50% bis zu 99.9% zugemischtes Lipid enthalten sind.

8. Partikuläre Wirkstoffträger nach einem der Ansprüche 1 bis 7, bei denen der LAK-Matrix ein oder mehrere Arzneistoffe zugemischt sind.

9. Partikuläre Wirkstoffträger nach Anspruch 8, bei denen der zugemischte Arzneistoff identisch ist mit dem im LAK an das Lipid gekoppeltem Arzneistoff.

10. Partikuläre Wirkstoffträger nach einem der Ansprüche 1 bis 9, bei denen das LAK durch Kopplung eines Arzneistoffes an ein oder mehrere Diglyceride, Monoglyceride, Glycerol, Fettsäuren, Fettalkohole, funktionelle Gruppen von Lipiden und Wachsen, insbesondere Sterolen, bevorzugt Cholesterol und Cholesterolderivate und Stigmasterin, einzeln oder in Mischung, hergestellt wurde.

11. Partikuläre Wirkstoffträger nach einem der Ansprüche 1 bis 10, bei denen das LAK als Arzneistoff Buttersäure enthält, und insbesondere Behenylalkohol-Buttersäure-Ester und Buttersäure-Triglycerid (Tributyrin) ist.

12. Partikuläre Wirkstoffträger nach einem der Ansprüche 1 bis 10, bei denen das LAK als Arzneistoff β-Aminosäure, ein Peptid oder ein Protein dieser Aminosäure enthält.

13. Partikuläre Wirkstoffträger nach einem der Ansprüche 1 bis 12, die durch Zerkleinern des Materials der Partikelmatrix mit Pulvermühlen (Trockenmahlung) hergestellt wurden, insbesondere durch Mahlung mit einer Mörsermühle, Kugelmühle, Schlagkreuzmühle oder Gasstrahlmühle.

14. Partikuläre Wirkstoffträger nach einem der Ansprüche 1 bis 12, die durch Zerkleinern des Materials der Partikelmatrix nach Aufschwämmung in einer wäßrigen oder nichtwäßrigen Flüssigkeit (Naßmahlung) hergestellt wurden, insbesondere durch eine Kolloid-Stator-Mühle (bevorzugt ein Ultra-Turrax oder ein Silverson Homogenisator), einen Kolben-Spalt-Hochdruckhomogenisatoren, eine Strömungsdispergiermaschine vom Typ Jet Stream (bevorzugt einen Mikrofluidizer) oder durch einen statischen Mischer im Mikromaßstab und im Makromaßstab (bevorzugt einen Sulzer-Mischer).

15. Partikuläre Wirkstoffträger nach einem der Ansprüche 1 bis 12, die durch Zerkleinern des Materials der Partikelmatrix im geschmolzenen oder teilgeschmolzenem Zustand nach Aufschwämmung in einer wäßrigen oder nichtwäßrigen Flüssigkeit (Naßmahlung) unter Verwendung der Methoden gemäß Anspruch 14 hergestellt wurden.

16. Partikuläre Wirkstoffträger nach einem der Ansprüche 1 bis 12, die durch Zerkleinern des Materials der Partikelmatrix im geschmolzenen oder teilgeschmolzenem Zustand durch Verteilen in einer Gasphase, insbesondere durch Versprühen des geschmolzenen Matrixmaterials in Luft, oder durch Verteilen in einer flüssigen Phase, insbesondere durch Verdüsung mittels Einstoffdüsen in Wasser, hergestellt wurden.

17. Partikuläre Wirkstoffträger nach einem der Ansprüche 1 bis 16, die in Form einer flüssigen Dispersion, festen Dispersion oder einer trockenen Form durch Flüssigkeitsentzug aus der flüssigen Dispersion (Sprühtrocknung, Lyophilisation, Walzentrocknung) vorliegen.

## Claims

1. Particulate vehicles for active ingredients (carriers), which are in the solid aggregate state at room temperature (20 °C), consisting of a pure lipid-drug conjugate (LDC) or a mixture of several LDCs as the particle matrix, wherein the bonding in the LDC is due to covalent bonds, electrostatic interactions, dipole-dipole interactions, dispersion forces, ion interactions, hydrogen bonds and/or hydrophobic interactions.

2. Particulate vehicles for active ingredients according to Claim 1, in which a proportion of covalent bonding is present in addition to electrostatic interactions, dipole-dipole interactions, dispersion forces, ion interactions, hydrogen bonds and/or hydrophobic interactions.

3. Particulate vehicles for active ingredients according to Claim 1 or 2 with an average particle size in the range from 10-1000 nanometres.

4. Particulate vehicles for active ingredients according to Claim 1 or 2 with an average particle size in the range from 10-1000 micrometres.

5. Particulate vehicles for active ingredients according to Claim 1 to 4, in which one or more lipids are added to the LDC matrix.

6. Particulate vehicles for active ingredients according to Claim 5, in which 0.1 % to 50% added lipid, relative to the overall weight, are comprised in the particle matrix.

7. Particulate vehicles for active ingredients according to Claim 5, in which 50% to 99.9% added lipid, relative to the overall weight, are comprised in the particle matrix.

8. Particulate vehicles for active ingredients according to one of Claims 1 to 7, in which one or more drugs are mixed into the LDC matrix.

9. Particulate vehicles for active ingredients according to Claim 8, in which the added drug is identical to the drug coupled to the lipid in the LDC.

10. Particulate vehicles for active ingredients according to one of Claims 1 to 9, in which the LDC was prepared by coupling a drug to one or more diglycerides, monoglycerides, glycerine, fatty acids, fatty alcohols, functional groups of lipids and waxes, particularly sterols, preferably cholesterol and cholesterol derivatives and stigmasterol, alone or in mixture.

11. Particulate vehicles for active ingredients according to one of Claims 1 to 10, in which the LDC comprises butyric acid as the drug, and is especially behenyl alcohol ester of butyric acid and butyric acid triglyceride (tributyrin).

12. Particulate vehicles for active ingredients according to one of Claims 1 to 10, in which the LDC comprises β-amino acid, a peptide or a protein of this amino acid as the drug.

13. Particulate vehicles for active ingredients according to one of Claims 1 to 12, which were prepared by comminution of the material of the particle matrix with powder mills (dry milling), especially by milling in a mortar grinder, ball mill, cross beater mill or gas jet mill.

14. Particulate vehicles for active ingredients according to one of Claims 1 to 12, which were prepared by comminution of the material of the particle matrix after suspension in an aqueous or non-aqueous liquid (wet milling), especially by means of a colloid stator mill (preferably an Ultra-Turrax or a Silverson homogeniser), a valve high pressure homogeniser, an air stream dispersion machine of the jet stream type (preferably a microfluidiser) or by a static mixer in the micro- or macro scale (preferably a Sulzer mixer).

15. Particulate vehicles for active ingredients according to one of Claims 1 to 12, which were prepared by comminution of the material of the particle matrix in the molten or partly molten state after suspension in an aqueous or non-aqueous liquid (wet milling) using the methods according to Claim 14.

16. Particulate vehicles for active ingredients according to one of Claims 1 to 12, which were prepared by comminution of the material of the particle matrix in the molten or partly molten state by dispersion in a gas phase, especially by spraying the molten matrix material in air, or by dispersion in a liquid phase, particularly by spraying using a one phase nozzle into water.

17. Particulate vehicles for active ingredients according to one of Claims 1 to 16, which are present in the form of a liquid dispersion, solid dispersion or a dried state by liquid removal from the liquid dispersion (spray drying, lyophilisation, roller drying).

## Revendications

1. Véhicules particulaires de substances actives, qui se trouvent en l'état d'agrégat solide à la température ambiante (20 °C), consistant en un Conjugué Lipide-Principe actif (CLP) pur ou en un mélange de plusieurs CLP sous forme de matrice particulaire, la liaison dans le CLP étant réalisée par liaison covalente, interactions électrostatiques, interactions dipôle-dipôle, forces de dispersion, interactions ioniques, ponts hydrogène et/ou interactions hydrophobes.

2. Véhicules particulaires de substances actives selon la revendication 1, dans lesquels, en plus d'interactions électrostatiques, d'interactions dipôle-dipôle, de forces de dispersion, d'interactions ioniques, de ponts hydrogène et/ou d'interactions hydrophobes, est également présente une proportion de liaison covalente.

3. Véhicules particulaires de substances actives selon la revendication 1 ou 2, ayant une taille moyenne de particule dans la plage de 10 à 1 000 nanomètres.

4. Véhicules particulaires de substances actives selon la revendication 1 ou 2, ayant une taille moyenne de particule dans la plage de 10 à 1 000 micromètres.

5. Véhicules particulaires de substances actives selon l'une quelconque des revendications 1 à 4, dans lesquels un ou plusieurs lipides sont ajoutés à la matrice CLP.

6. Véhicules particulaires de substances actives selon la revendication 5, dans lesquels de 0,1 % à 50 % de lipide ajouté sont contenus dans la matrice particulaire, par rapport au poids total.

7. Véhicules particulaires de substances actives selon la revendication 5, dans lesquels de 50 % à 99,9 % de lipide ajouté sont contenus dans la matrice particulaire, par rapport au poids total.

8. Véhicules particulaires de substances actives selon l'une quelconque des revendications 1 à 7, dans lesquels un ou plusieurs principes actifs sont ajoutés à la matrice CLP.

9. Véhicules particulaires de substances actives selon la revendication 8, dans lesquels le principe actif ajouté est identique au principe actif conjugué au lipide dans le CLP.

10. Véhicules particulaires de substances actives selon l'une quelconque des revendications 1 à 9, dans lesquels le CLP a été préparé par couplage d'un principe actif à un ou plusieurs diglycérides, monoglycérides, au glycérol, à un ou plusieurs acides gras, alcools gras, groupes fonctionnels de lipides et de cires, en particulier à des stérols, de préférence au cholestérol et à des dérivés de cholestérol et à la stigmastérine, seuls ou en mélange.

11. Véhicules particulaires de substances actives selon l'une quelconque des revendications 1 à 10, dans lesquels le CLP contient en tant que principe actif de l'acide butyrique, en particulier du butyrate de béhényle et du triglycéride d'acide butyrique (tributyrine).

12. Véhicules particulaires de substances actives selon l'une quelconque des revendications 1 à 10, dans lesquels le CLP contient en tant que principe actif un acide β-aminé, un peptide ou une protéine de cet acide aminé.

13. Véhicules particulaires de substances actives selon l'une quelconque des revendications 1 à 12, qui ont été préparés par fragmentation de la matière de la matrice particulaire à l'aide de broyeurs à poudre (broyage à sec), en particulier par broyage au moyen d'un broyeur à mortier, d'un broyeur à billes, d'un broyeur à marteaux fixes en croix ou d'un broyeur à jet de gaz.

14. Véhicules particulaires de substances actives selon l'une quelconque des revendications 1 à 12, qui ont été préparés par fragmentation de la matière de la matrice particulaire après mise en suspension dans un liquide aqueux ou non aqueux (broyage par voie humide), en particulier dans un broyeur-stator à colloïde (de préférence un homogénéisateur Ultra-Turrax ou un homogénéisateur Silverson), un homogénéisateur piston haute pression à fente, une machine de dispersion à flux du type Jet Stream (de préférence un microfluidiseur) ou dans un mélangeur statique à petite et à grande échelle (de préférence un mélangeur Sulzer).

15. Véhicules particulaires de substances actives selon l'une quelconque des revendications 1 à 12, qui ont été obtenus par fragmentation de la matière de la matrice particulaire à l'état fondu ou partiellement fondu après mise en suspension dans un liquide aqueux ou non aqueux (broyage par voie humide) à l'aide des méthodes selon la revendication 14.

16. Véhicules particulaires de substances actives selon l'une quelconque des revendications 1 à 12, qui ont été obtenus par fragmentation de la matière de la matrice particulaire à l'état fondu ou partiellement fondu par répartition dans une phase gazeuse, en particulier par pulvérisation de la matière fondue de la matrice dans l'air ou par répartition dans une phase liquide, en particulier par pulvérisation dans de l'eau au moyen de buses monocomposant.

17. Véhicules particulaires de substances actives selon l'une quelconque des revendications 1 à 16, qui se trouvent sous forme d'une dispersion liquide, d'une dispersion solide ou sous une forme sèche, par extraction du liquide de la dispersion liquide (séchage par atomisation, lyophilisation, séchage sur un sécheur à cylindres).
